(19) Europäisches Patentamt
European Patent Office
Office européen des brevets

(11) Veröffentlichungsnummer: **0 043 054**
**B1**

# (12) EUROPÄISCHE PATENTSCHRIFT

(45) Veröffentlichungstag der Patentschrift: **03.10.84**

(21) Anmeldenummer: **81104750.5**

(22) Anmeldetag: **22.06.81**

(51) Int. Cl.³: **C 07 D 495/14,** A 61 K 31/505 // C07D333/38, C07D495/04, (C07D495/14, 333/00, 239/00, 235/00)

## (54) Neue Pyrimidinderivate, ihre Herstellung und Arzneimittel enthaltend diese Derivate.

(30) Priorität: **27.06.80 CH 4995/80**

(43) Veröffentlichungstag der Anmeldung: **06.01.82 Patentblatt 82/1**

(45) Bekanntmachung des Hinweises auf die Patenterteilung: **03.10.84 Patentblatt 84/40**

(84) Benannte Vertragsstaaten: **AT BE CH DE FR GB IT LI LU NL SE**

(56) Entgegenhaltungen:
**EP - A - 0 008 408**
**DE - A - 2 411 273**
**DE - A - 2 411 274**

(73) Patentinhaber: **F. HOFFMANN-LA ROCHE & CO. Aktiengesellschaft, CH-4002 Basel (CH)**

(72) Erfinder: **Kienzle, Frank, Dr., Tannwaldweg 9, CH-4113 Flüh (CH)**

(74) Vertreter: **Lederer, Franz, Dr. et al, Patentanwälte Lederer, Meyer Lucile-Grahn-Strasse 22, D-8000 München 80 (DE)**

**EP 0 043 054 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Pyrimidinderivate der Formel

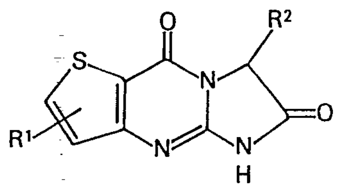

I

worin $R^1$ Wasserstoff, Chlor, Brom oder $C_{1-4}$-Alkyl und $R^2$ Wasserstoff oder Methyl sind.

Der hier verwendete Ausdruck $C_{1-4}$-Alkyl bezieht sich auf geradkettige und verzweigte Reste, wie Methyl, Aethyl, Propyl und t-Butyl.

Beispiele von Verbindungen der Formel I sind Imidazo [1,2-a] thieno [3,2-d] pyrimidin-6,9 (5H,7H)-dion, 7-Mehtyl-imidazo[1,2-a] thieno [3,2-d] pyrimidin-6,9 (5H,7H)-dion und

2- oder 3- (Methyl oder Chlor)-imidazo[1,2-a] thieno [3,2-d] pyrimidin-6,9 (5H,7H)-dion, wobei die erstgenannte bevorzugt ist.

Die Erfindung betrifft weiterhin ein Verfahren zur Herstellung der Verbindungen I, sowie pharmazeutische Präparate auf der Basis dieser Verbindungen.

Die Verbindungen der Formel I können erfindungsgemäss dadurch hergestellt werden, dass man einen Ester der Formel

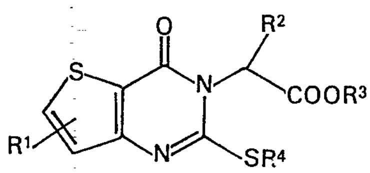

II

worin $R^1$ und $R^2$ die obige Bedeutung haben und $R^3$ und $R^4$ $C_{1-4}$-Alkyl darstellen, mit Ammoniak umsetzt.

Der Ester II kann mit einer Lösung von Ammoniak in Wasser oder in einem Alkohol, z.B. einem Alkanol, wie Methanol, bei einer Temperatur von 0–150°C, vorzugsweise bei etwa 120°C, zweckmässigerweise unter Druck umgesetzt werden. Die Umsetzung kann auch mit flüssigem Ammoniak vorgenommen werden.

Die Verbindungen der Formel I können in verschiedenen tautomeren Formen vorliegen. Die Erfindung beschränkt sich daher nicht auf Verbindungen der oben dargestellten Formel I, sondern umfasst auch die Tautomeren, z.B. solche der Formeln

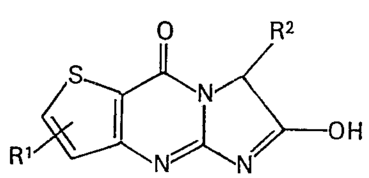

und

Ia

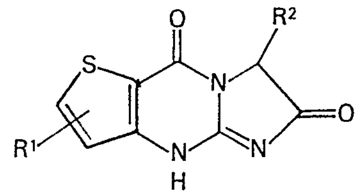

Ib

Die Verbindungen der Formel I, worin $R^2$ Methyl ist, und deren Tautomeren können weiterhin in Form von Racematen oder in optisch aktiver Form vorliegen, wobei alle diese Formen Gegenstand der Erfindung sind.

Die Ester der Formel II können durch Umsetzung einer Verbindung der Formel

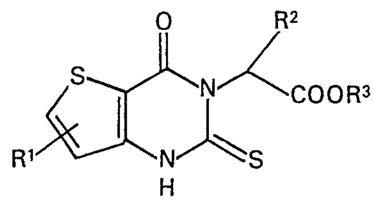

III

mit einem Dialkylsulfat der Formel $(R^4O)_2SO_2$ oder einem Halogenid der Formel $R^4Hal$, worin $R^1$, $R^2$, $R^3$ und $R^4$ die obige Bedeutung haben und Hal Halogen, vorzugsweise Jod, darstellt, hergestellt werden. Zweckmässigerweise wird die Reaktion in Gegenwart einer Base, z.B. einem Alkalimetallcarbonat, wie Kaliumcarbonat, in einem aprotischen Lösungsmittel, wie DMF, DMSO oder, bei Verwendung eines Dialkylsulfats, auch Aceton, bei einer Temperatur von 0°C bis zu Rückflusstemperatur des Reaktionsgemisches, vorzugsweise unter Erhitzen, durchgeführt.

Die Verbindungen III können durch Umsetzung einer Verbindung der Formel

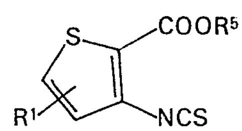

IV

mit einem Ester der Formel $H_2N\text{-}\overset{\overset{R^2}{|}}{C}H\text{-}COOR^3$, worin $R^1$, $R^2$ und $R^3$ die obigen Bedeutungen haben und $R^5$ $C_{1-4}$-Alkyl darstellt, hergestellt werden. Zweckmässigerweise wird die Reaktion in Gegenwart einer Base, z.B. einem Trialkylamin, wie Triäthylamin, in einem aprotischen Lösungsmittel, wie THF, Benzol oder Pyridin, bei einer Temperatur bis zu etwa 100°C, vorzugsweise bei Raumtemperatur, durchgeführt.

Die Verbindungen IV können durch Umsetzung einer Verbindung der Formel

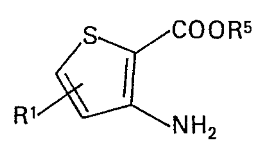

V

worin $R^1$ und $R^5$ die obigen Bedeutungen haben, mit Thiophosgen, zweckmässigerweise in Gegenwart einer Base, z.B. einem Alkalimetallcarbonat, wie Natriumcarbonat, oder einem Trialkylamin, wie Triäthylamin, in einem apolaren Lösungsmittel, wie Chloroform oder Tetrachlorkohlenstoff, bei einer Temperatur zwischen etwa −20 und 0°C, hergestellt werden.

In den Verbindungen der Formeln II, III, IV und V sind $R^3$, $R^4$ und $R^5$ vorzugsweise Methyl.

Die Verbindungen der Formel II und III sind neu und als solche zur Verwirklichung der vorliegenden Erfindung notwendig.

Die Verbindungen der Formel I sind Heilmittel, insbesondere zur Verhütung von allergischen Reaktionen, z.B. zur prophylaktischen Behandlung von Bronchialasthma. Die antiallergische Wirksamkeit wird durch das folgende Experiment demonstriert:

Männliche Ratten wurden durch eine i.V. Injektion von Antiovalbuminantikörper enthaltendem Plasma (1 ml pro Tier) sensibilisiert. Eine anaphylaktische Bronchialasthmareaktion wurde bei Bewusstsein 18 Std. später durch eine i.v. Injektion von Ovalbumin 5 mg pro kg) ausgelöst. Als Mass für diese Reaktion wurden die Atmungsfrequenz und das Verhältnis zwischen Exspirations- und Inspirationszeit mit Hilfe eines Pneumographen gemessen. Die Testsubstanzen wurden p.o., im Falle des Imidazo [1,2-a] thieno [3,2-d] pyrimidin-6,9 (5H,7H)-dions ½ Std. vor der Injektion des Ovalbumins, verabreicht. Als Mass für die Wirksamkeit wurde die Fähigkeit der Testsubstanz, die Atmungsfrequenz und das Verhältnis Exspirationszeit zur Inspirationszeit zu verkleinern, genommen. Dabei wurde für das obige Pyrimidinderivat eine $ED_{50}$ von 32 mg /kg gefunden. Die Testresultate sind in der nachstehenden Tabelle wiedergegeben:

| Dosis mg pro kg | Atmungs-frequenz | Abnahme % | Verhältnis Exspirationszeit/ Inspirationszeit | Abnahme % |
|---|---|---|---|---|
| 12,5 | 97,4 ± 13,7 | 32,1 | 403,9 ± 94,9 | 73,6 |
| 25 | 88,5 ± 20,7 | 38,0 | 357,5 ± 86,1 | 40,1 |
| 50 | 71,3 ± 53,9 | 53,9 | 210,7 ± 68,1 | 64,7 |

Die Verbindungen der Formel I können enteral, insbesondere oral, oder parenteral als antiallergische Mittel, z.B. für prophylaktische Behandlung von Bronchialasthma, in den individuellen Erfordernissen angepassten Dosen verabreicht werden. Sie können therapeutisch, z.B. enteral, insbesondere oral, oder parenteral, durch Einverleibung einer therapeutischen Dosis in einer üblichen Dosisform, wie Tabletten, Kapseln, Elixiere, Suspensionen oder Lösungen verabreicht werden. Sie können in Mischung mit üblichen pharmazeutischen Trägern oder Bindemitteln, wie Maisstärke, Kalziumstearat, Magnesiumkarbonat, Kalziumsilikat, Dikalziumphosphat, Talk oder Laktose verabreicht werden. Darüber hinaus können sie in Gegenwart von Puffern oder zur Einstellung der Isotonie verwendeten Mitteln verabreicht werden, und die pharmazeutischen Dosisformen können, wenn erwünscht, den üblichen pharmazeutischen Massnahmen, wie Sterilisation, unterworfen werden. Sie können auch andere therapeutisch wertvolle Substanzen enthalten.

Die Menge an Wirkstoff, welche in irgendeiner der oben beschriebenen Dosisformen vorhanden ist, ist variabel. Kapseln oder Tabletten können z.B. etwa 10 mg bis etwa 20 mg einer Verbindung der Formel I enthalten.

Die Häufigkeit, mit der eine solche Dosisform an einen Patienten verabreicht wird, schwankt und ist von der in der Dosisform vorhandenen Menge an wirksamem Medikament und den Notwendigkeiten und Erfordernissen des Patienten abhängig. Unter normalen Umständen können jedoch von der Verbindung bis zu etwa 20 mg/kg täglich in mehreren Dosen verabreicht werden. Es ist jedoch selbstverständlich, dass die angegebenen Dosen nur beispielhaft sind.

Die folgenden Beispiele erläutern die Erfindung weiter. Alle Temperaturen sind in Grad Celsius angegeben.

Beispiel 1

Ein Gemisch von 15 g 2-Methylthio-4-oxothieno [3,2-d] pyrimidin-3(4H)-essigsäuremethylester und 300 ml bei −10° mit Ammoniak gesättigtem Methanol wird 90 Std. bei 120° und 28 Atü gerührt. Nach dem Abkühlen werden nicht gelöste Anteile abfiltriert und das Filtrat wird mit Salzsäure sauer gestellt. Das Produkt wird dann abfiltriert und aus Eisessig umkristallisiert. Ausbeute: 7g Imidazo [1,2-a]thieno [3,2-d]pyrimidin-6,9(5H,7H)-dion, Smp. > 250°; IR (in KBr): 3094 (s), 2746 (s), 1785 (s), 1764 (s), 1700 (s), 1644 (s), 1517 (s), 1440 (s), 1315 (m), 1219 (s), 1145 (m), 1049 (m), 790 (m), 732 (m). Das Ausgangsmaterial kann wie folgt hergestellt werden:

Zu einem bei 0°C gerührten Gemisch von 320 ml Chloroform, 120 ml Wasser und 55,4 g Natriumcarbonat werden 20 ml Thiophosgen getropft, gefolgt im Laufe von 15 Min. von 38,7 g 3-Amino-2-thiophencarbonsäuremethylesterhydrochlorid. Dann wird 90 Min. bei Raumtemperatur gerührt. Die organische Phase wird abgetrennt, getrocknet und eingedampft. Der Rückstand wird aus Chloroform-Pentan umkristallisiert. Ausbeute: 42,5 g 3-Isothiocyanato-2-thiophencarbonsäuremethylester, Smp. 60–61°C.

Ein Gemisch von 38 g Glycinmehtylester, 380 ml Tetrahydrofuran und 40 ml Triäthylamin wird bei Raumtemperatur mit einer Lösung von 42,5 g 3-Isothiocyanato-2-thiophencarbonsäuremethylester versetzt und 20 Std. bei Raumtemperatur gerührt. Dann wird auf Wasser gegossen und das ausfallende produkt abfiltriert. Ausbeute 46,5 g 1,4-Dihydro-4-oxo-2-thioxothieno [3,2-d] pyrimidin-3(2H)-essigsäuremethylester, Smp. 240° (Zers.).

Ein Gemisch von 46,5 g 1,4-Dihydro-4-oxo-2-thioxothieno [3,2-d]pyrimidin-3(2H)-essigsäuremethylester, 875 ml Aceton und 110 g Kaliumcarbonat wird mit 45 ml Dimethylsulfat versetzt und 3 Std. unter Rückfluss gekocht. Dann wird auf Wasser gegossen und das Produkt abfiltriert. Ausbeute nach Umkristallisation aus Essigester: 36 g 2-Methylthio-4-oxothieno[3,2-d]pyrimidin-3(4H)-essigsäuremehtylester, Smp. 167–169°C.

Beispiel 2

In zu Beispiel 1 analoger Weise werden ausgehend von 30 g 2-Methylthio-7-methyl-4-oxothieno[3,2-d]pyrimidin-3(4H)-essigsäuremehtylester 15 g 3-Methylimidazo[1,2-a]-thiono[3,2-d]pyrimidin-6,9(5H,7H)-dion, Smp. 212° (Zers.) hergestellt.

Das Ausgangsmaterial kann in zu Beispiel 1 analoger Weise ausgehend von 3-Amino-4-methyl - 2 - thiophencarbonsäuremethylesterhydrochlorid über 4-Methyl-3-isothiocyanato-2-thiophencarbonsäuremethylester (70 g, Smp. 227–228°) und 1,4-Dihydro-7-methyl-4-oxo-2-thioxothieno[3,2-d]pyrimidon-3(2H)-essigsäuremethylester (69 g, Smp. 227–228°) hergestellt werden; Ausbeute 68,9 g, Smp. 131–132°.

Beispiel 3

| Kapselformulierung | mg/Kapsel 10 mg | 20 mg |
|---|---|---|
| Wirkstoff der Formel I | 10,0 | 20 |
| Laktose | 215,0 | 205,0 |
| Maisstärke | 60,0 | 60,0 |
| Magnesiumstearat | 3,0 | 3,0 |
| Talk | 12,0 | 12,0 |
| Total | 300,0 mg | 300,0 mg |

Verfahren: Der Wirkstoff der Formel I, Laktose und Maisstärke werden in einem geeigneten Mischer gemischt. Es wird durch eine geeignete Mühle gemahlen, mit Magnesiumstearat und Talk gemischt und auf einer Kapselmaschine gefüllt.

Beispiel 4

| Tablettenformulierung: | mg/Tablette 10 mg | 20 mg |
|---|---|---|
| Wirkstoff der Formel I | 10,0 | 20,0 |
| Laktose | 182,0 | 172,0 |
| Mikrokristalline Cellulose | 60,0 | 60,0 |
| Modifizierte Stärke | 15,0 | 15,0 |
| Maisstärke | 30,0 | 30,0 |
| Magnesiumstearat | 3,0 | 3,0 |
| Total | 300,0 mg | 300,0 mg |

Verfahren: Der Wirkstoff der Formel I, Laktose, mikrokristalline Cellulose, modifizierte Stärke und Maisstärke, wird in einem geeigneten Mischer 1 bis 15 Min. gemischt. Dann wird Magnesiumstearat zugefügt und 5 Min gemischt. Auf einer geeigneten Presse wird verpresst.

Beispiel 5

| Nassgranulierte Tablettenformulierung | mg/Tablette 10 mg | 20 mg |
|---|---|---|
| Wirkstoff der Formel I | 10,0 | 20,0 |
| Laktose | 264,0 | 254,0 |
| Vorgelatinierte Stärke | 17,5 | 17,6 |
| Maisstärke | 35,0 | 35,0 |
| Modifizierte Stärke | 17,5 | 17,5 |
| Magnesiumstearat | 6,0 | 6,0 |
| Total | 350,0 mg | 350,0 mg |

Verfahren: Der Wirkstoff der Formel I, Laktose und vorgelatinierte Stärke, wird in einem geeignetem Mischer vermischt. Es wird durch eine geeignete Mühle gemahlen, mit modifizierter Stärke und Magnesiumstearat gemischt und auf einer Kapselmaschine gefüllt.

**Patentansprüche für die Vertragsstaaten: BE, CH, DE, FR, GB, IT, LI, LU, NL, SE.**

1. Pyrimidinderivate der Formel

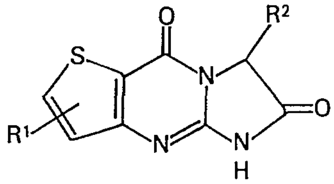

I

worin $R^1$ Wasserstoff, Chlor, Brom oder $C_{1-4}$-Alkyl und $R^2$ Wasserstoff oder Methyl sind, und deren Tautomere.

2. Imidazo[1,2-a]thieno[3,2-d]pyrimidin-6,9 (5H,7H)-dion.

3. 3-Methylimidazo[1,2-a]thieno[3,2-d]pyrimidin-6,9(5H,7H)-dion.

4. Eine Verbindung nach Anspruch 1, 2 oder 3 zur Anwendung als pharmazeutischer Wirkstoff.

5. Eine Verbindung nach Anspruch 1, 2 oder 3 zur Anwendung bei der Behandlung bzw. Verhütung von allergischen Reaktionen.

6. Pharmazeutische Präparate enthaltend eine Verbindung nach einem der Ansprüche 1–3.

7. Antiallergisches Präparat gemäss Anspruch 6.

8. Verfahren zur Herstellung von Verbindungen gemäss Anspruch 1, dadurch gekennzeichnet, dass man einen Ester der Formel

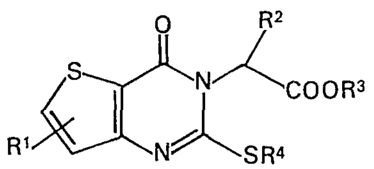

II

worin $R^1$ und $R^2$ die obige Bedeutung haben und $R^3$ und $R^4$ $C_{1-4}$-Alkyl darstellen, mit Ammoniak umsetzt.

**Patentansprüche für den Vertragsstaat: AT**

1. Verfahren zur Herstellung von Pyrimidinderivaten der Formel

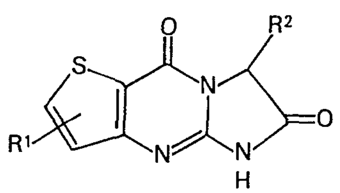

I

worin $R^1$ Wasserstoff, Chlor, Brom oder $C_{1-4}$-Alkyl und $R^2$ Wasserstoff oder Methyl sind, und deren Tautomeren dadurch gekennzeichnet, dass man einen Ester der Formel

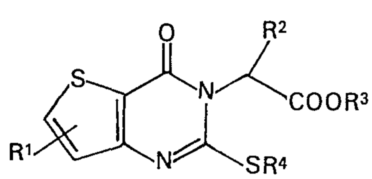

II

worin $R^1$ und $R^2$ die obige Bedeutung haben und $R^3$ und $R^4$ $C_{1-4}$-Alkyl darstellen, mit Ammoniak umsetzt.

2. Verfahren nach Anspruch 1, dadurch gekennzeichnet, dass man Imidazo[1,2-a]thieno[3,2-d]pyrimidon-6,9(5H,7H)-dion herstellt.

3. Verfahren nach Anspruch 1, dadurch gekennzeichnet, dass man 3-Methylimidazo[1,2-a]thieno[3,2-d]pyrimidin6,9(5H,7H)-dion herstellt.

**Claims for the Contracting States: BE, CH, DE, FR, GB, IT, LI, LU, NL, SE.**

1. Pyrimidine derivatives of the formula

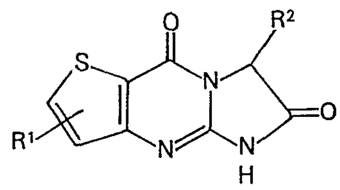

I

wherein $R^1$ is hydrogen, chlorine, bromine or $C_{1-4}$-alkyl and $R^2$ is hydrogen or methyl, and their tautomers.

2. Imidazo[1,2-a]thieno[3,2-d]pyrimidine-6,9(5H,7H)-dione.

3. 3-Methylimidazo[1,2-a]thieno[3,2-d]pyrimidine-6,9(5H,7H)-dione.

4. A compound according to claim 1, 2 or 3 for use as a pharmaceutically active substance.

5. A compound according to claim 1, 2 or 3 for use in the treatment or prevention of allergic reactions.

6. Pharmaceutical preparations containing a compound according to any one of claims 1–3.

7. An antiallergic preparation in accordance with claim 6.

8. A process for the manufacture of compounds in accordance with claim 1, characterized by reacting an ester of the formula

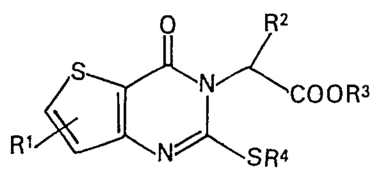

II

wherein $R^1$ and $R^2$ have the above significance and $R^3$ and $R^4$ represent $C_{1-4}$-alkyl, with ammonia.

**Claims for the Contracting State: AT**

1. A process for the manufacture of pyrimidine derivatives of the formula

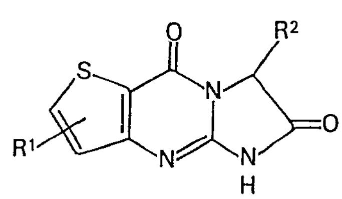

I

wherein $R^1$ is hydrogen, chlorine, bromine or $C_{1-4}$-alkyl and $R^2$ is hydrogen or methyl,

and their tautomers, characterized by reacting an ester of the formula

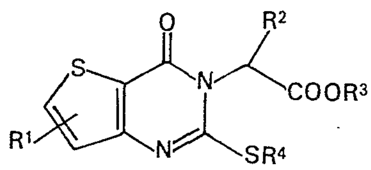

II

wherein $R^1$ and $R^2$ have the above significance and $R^3$ and $R^4$ represent $C_{1-4}$-alkyl, with ammonia.

2. A process according to claim 1, characterized in that imidazo[1,2-a]thieno[3,2-d]pyrimidine-6,9(5H,7H)-dione is manufactured.

3. A process according to claim 1, characterized in that 3-methylimidazo[1,2-a]thieno[3,2-d] pyrimidine-6,9-(5H,7H)-dione is manufactured.

**Revendications pour les Etat contractants: BE, CH, DE, FR, GB, IT, LI, LU, NL, SE**

1. Dérivés de pyrimidine de formule

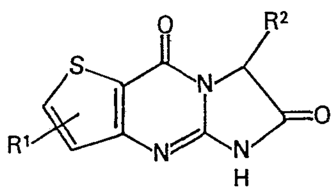

I

où $R^1$ représente l'hydrogène, le chlore, le brome ou un groupe alkyle en $C_1$ à $C_4$ et $R^2$ représente l'hydrogène ou un groupe méthyle, et leurs tautomères.

2. Imidazo[1,2-a]thiéno[3,2-d]pyrimidine-6,9 (5H,7H)-dione.

3. 3-méthylimidazo[1,2-a]thiéno[3,2-d]pyrimidine-6,9-(5H,7H)-dione.

4. Composé selon l'une des revendications 1, 2 ou 3 aux fins d'application comme substance active pharmaceutique.

5. Composés selon l'une des revendications 1, 2 ou 3 aux fins d'application dans le traitement ou selon les cas la prévention des réactions allergiques.

6. Préparations pharmaceutiques contenant un composé selon l'une des revendications 1-3.

7. Préparation anti-allergique selon la revendication 6.

8. Procédé de préparation de composés selon la revendication 1, caractérisé en ce qu'on fait réagir un ester de formule

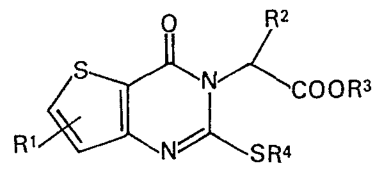

II

où $R^1$ et $R^2$ ont la signification donnée ci-dessus et $R^3$ et $R^4$ représentent des groupes alkyles en $C_1$ à $C_4$, avec de l'ammoniac.

**Revendications pour l'Etat contractant: AT**

1. Procédé de préparation de dérivés de pyrimidine de formule

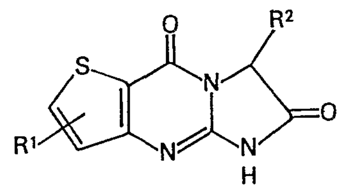

I

où $R^1$ représente l'hydrogène, le chlore, le brome ou un groupe alkyle en $C_1$ à $C_4$ et $R^2$ représente un hydrogène ou un méthyle,

et de leurs tautomères, caractérisé en ce qu'on fait réagir un ester de formule

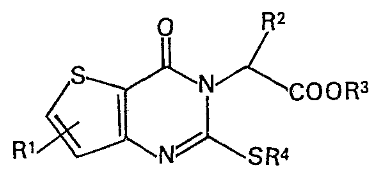

II

où $R^1$ et $R^2$ ont les significations données ci-dessus et $R^3$ et $R^4$ représentent des groupes alkyles en $C_1$ à $C_4$, avec de l'ammoniac.

2. Procédé selon la revendication 1, caractérisé en ce qu'on prépare l'imidazo[1,2-a]thiéno [3,2-d]pyrimidine-6,9(5H,7H)-dione.

3. Procédé selon la revendication 1, caractérisé en ce qu'on prépare la 3-méthylimidazo [1,2-a]thiéno[3,2-d]-pyrimidine-6,9(5H,7H)-dione.